# EUROPEAN PATENT APPLICATION

(11) **EP 4 372 381 A1**
(43) Date of publication of application: **22.05.2024**
(21) Application number: 22816097.4
(22) Date of filing: 31.05.2022
(51) Int. Cl.: G01N 33/68, G01N 33/92

(54) **NOVEL METHOD FOR MEASURING CHOLESTEROL EFFLUX CAPACITY**

(30) Priority: 04.06.2021 JP 2021094456
(71) Applicant: National University Corporation Tokyo Medical and Dental University, Tokyo 113-8510 (JP)
(72) Inventor: OHKAWA Ryunosuke, Tokyo 113-8510 (JP); MUTSUDA Yume, Tokyo 113-8510 (JP); HORIUCHI Yuna, Tokyo 113-8510 (JP); KAMEDA Takahiro, Tokyo 113-8510 (JP); TOZUKA Minoru, Tokyo 113-8510 (JP)
(74) Representative: Plasseraud IP
(86) International application number: PCT/JP2022/022081
(87) International publication number: WO 2022/255351

(57) **Abstract**

The present invention provides a novel method for measuring cholesterol efflux capacity. The present invention provides a method for measuring the cholesterol efflux capacity of HDL, the method including: a step for preparing magnetic beads obtained by converting cholesterol to a solid phase; a step for bringing the magnetic beads obtained by converting cholesterol to a solid phase into contact with an HDL-containing sample to be measured in a solution; and a step for separating the magnetic beads from the solution and measuring the amount of cholesterol in the solution after separating the magnetic beads.

## Description

### Technical Field

The present invention relates to a novel method for measuring cholesterol efflux capacity, and particularly to a method for measuring cholesterol efflux capacity using magnetic beads.

### Background Art

Currently, measurement of high-density lipoprotein cholesterol (HDL-C) concentration is widely performed as an indicator of dyslipidemia. However, HDL is a heterogeneous particle in terms of structure, composition, biological activity, and such; and there is a problem that only in some of the patients, the accurate state of lipid abnormalities can be reflected by the method of evaluating the total amount of HDL by cholesterol quantification.

In recent years, research that evaluates the quality (function) of HDL in addition to its quantity has attracted worldwide attention. For example, a representative function of HDL is the reverse cholesterol transport (RCT), which transports excessively accumulated cholesterol in foam cells that form an atherosclerotic lesion under the arterial endothelium to the liver. It has been proposed to assess cholesterol efflux capacity (CEC) as the ability of HDL to extract cholesterol from macrophages, which is a major step in the reverse transport system. For example, Non-Patent Document 1 suggests that cholesterol efflux capacity is useful as a biomarker independent of HDL-C concentration in cohort studies, and that CEC is inversely correlated with the onset of cardiovascular events, and the like.

As a method for measuring the ability to extract cholesterol (CEC evaluation method), a method that uses cultured cells (macrophages) that have taken up cholesterol labeled with a radioactive substance or a fluorescent substance is known (Non-Patent Document 2). However, since such previously reported CEC evaluation methods use cultured cells and radioactive materials, there is the problem that it is difficult to implement them into routine examinations in clinical practice.

On the other hand, in Non-Patent Document 3, as a CEC evaluation method that does not use radiolabeled cholesterol and cultured macrophages, a method of evaluating cholesterol uptake capacity using fluorescently labeled cholesterol and anti-apolipoprotein A1 antibody is proposed. In this method, (i) HDL is incubated with BODIPY-labeled cholesterol, (ii) HDL containing BODIPY-labeled cholesterol is captured with a specific antibody (anti-apoA1 antibody) immobilized on a microplate, and (iii) the fluorescence intensity of the labeled cholesterol incorporated into the HDL is measured (Fig. 1). That is, the method of Non-Patent Document 3 is a method of evaluating the ability of HDL to take up labeled free cholesterol in solution.

On the other hand, the present inventors have developed a method of using immobilized liposome-bound gel beads (ILG) as a new CEC measurement method that does not use radioactive substances or cultured cells used in conventional CEC evaluation methods (Non-Patent Document 4). In this method, liposome-bound gel beads, in which liposomes are prepared by adding a small amount of fluorescence-labeled cholesterol to lecithin and cholesterol and immobilized on gel beads, are incubated with apoB-depleted serum (BDS) as a cholesterol acceptor, followed by centrifugation to separate the liposome-bound gel beads, and the fluorescence intensity in the supernatant is measured. That is, this method is a method for directly evaluating the ability of HDL to extract cholesterol. The present inventors have revealed that this new CEC measurement method of using liposome-bound gel beads (ILG) shows a good correlation with the conventional CEC evaluation method of using [³H]cholesterol and macrophages differentiated from THP-1 cells. In addition, Non-Patent Document 5 shows that apoB deleted serum (BDS), which is easy to prepare, can be evaluated as a cholesterol acceptor in the CEC measurement method of using liposome-bound gel beads, in the same manner as HDL.

### Citation List

### Non-Patent Documents

Non-Patent Document 1: Rohatgi A et al. HDL cholesterol efflux capacity and incident cardiovascular events. N Engl J Med. 2014;371(25):2383-93.
Non-Patent Document 2: Sankaranarayanan et al. J Lipid Res, 2011;52:2332-40
Non-Patent Document 3: A. Harada et al. Cholesterol uptake capacity: A new measure of hdl functionality for coronary risk assessment. J. Appl. Lab. Med. 2017;2(2):186-200
Non-Patent Document 4: Horiuchi Y et al. Validation and application of a novel cholesterol efflux assay using immobilized liposomes as a substitute for cultures cells. Biosci Rep. 2018;38(2): BSR20180144
Non-Patent Document 5: Horiuchi Y et al. Usefulness of apolipoprotein B-depleted serum in cholesterol efflux capacity assay using immobilized liposome-bound gel beads. Biosci Rep. 2019;39(4): BSR20190213

### Summary of the Invention

### Problems to be Solved by the Invention

However, in the CEC evaluation method of using liposome-bound gel beads (ILG), since it is necessary to separate the gel beads by centrifugation, it is difficult to fully automate the process from sample collection to measurement, and there remains the problem that further development is required for automation (installation on automatic analyzer).

The present invention has been made in view of the above issues, and the objective is to provide a method for measuring the ability of HDL to extract cholesterol that is clinically applicable and easily applied to an automatic analyzer.

### Means for Solving the Problems

The present invention relates to the embodiments below.

### Embodiment 1

A method for measuring the cholesterol efflux capacity of HDL, wherein the method comprises: preparing magnetic beads on which cholesterol is immobilized;
contacting the magnetic beads on which cholesterol is immobilized with an HDL-containing sample to be measured in a solution; and separating the magnetic beads from the solution and measuring the amount of cholesterol in the solution after separation of the magnetic beads.

### Embodiment 2

The method according to embodiment 1, wherein the magnetic beads are porous magnetic particles.

### Embodiment 3

The method according to embodiment 1 or 2, wherein the step of preparing magnetic beads on which cholesterol is immobilized comprises preparing liposomes comprising cholesterol and freezing and thawing a solution comprising the liposomes and magnetic beads.

### Embodiment 4

The method according to any one of embodiments 1 to 3, wherein at least part of the cholesterol immobilized on the magnetic beads is fluorescently labeled.

### Embodiment 5

The method according to any one of embodiments 1 to 4 , which is a method for measuring the cholesterol efflux capacity of HDL, comprising:
preparing magnetic beads on which cholesterol is immobilized by preparing liposomes comprising at least partially labeled cholesterol, and freezing and thawing a solution comprising the liposomes and porous magnetic particles;
contacting the magnetic beads on which cholesterol is immobilized with an HDL-containing sample to be measured in a solution; and
separating the magnetic beads from the solution and measuring the amount of cholesterol in the solution after magnetic bead separation based on the labeling intensity of the solution.

### Embodiment 6

The method according to embodiment 5, wherein the cholesterol-immobilized magnetic beads have storage stability represented by the fact that the labeling intensity of a suspension medium collected from a suspension of the magnetic beads does not substantially change for at least 10 days.

### Embodiment 7

The method according to any one of embodiments 1 to 6, wherein the magnetic beads are magnetic ceramic particles.

### Embodiment 8

The method according to any one of embodiments 1 to 7, wherein the step of separating the magnetic beads from the solution does not include the operation of centrifugation.

### Embodiment 9

The method according to any one of embodiments 1 to 8, further comprising performing blank correction by subtracting the measured value of cholesterol quantity in the blank sample from the measured value of cholesterol quantity in the HDL-containing sample to be measured.

### Embodiment 10

The method according to embodiment 9, which does not include washing the magnetic beads on which cholesterol is immobilized before the step of contacting the magnetic beads on which cholesterol is immobilized with the HDL-containing sample to be measured in a solution.

### Embodiment 11

The method according to any one of embodiments 1 to 10, wherein the HDL-containing sample to be measured is whole blood, serum, plasma, apoB-depleted serum, or a solution comprising purified HDL.

### Embodiment 12

The method according to any one of embodiments 1 to 11, which is performed in a cell-free system.

### Embodiment 13

The method according to any one of embodiments 1 to 12, which has a reproducibility represented by a coefficient of variation of 10% or less for cholesterol quantity measurements of the same sample over at least 10 days.

### Embodiment 14

A reagent kit for measuring the cholesterol efflux capacity of HDL, comprising magnetic beads on which cholesterol is immobilized.

### Embodiment 15

A method for obtaining information on a condition related to the cholesterol efflux capacity of a subject or risk thereof, comprising measuring the cholesterol efflux capacity of an HDL-containing sample derived from the subject using magnetic beads on which cholesterol is immobilized.

### Effect of the Invention

According to the present invention, a method for measuring the cholesterol efflux capacity of HDL, which is clinically applicable and easily applicable to automatic analyzers, is provided.

### Brief Description of the Drawings

[Figure 1] It is a graph showing the relationship between the amount of fluorescent liposome immobilized on magnetic beads and the number of freeze-thaw cycles in the Examples.
[Figure 2] It is a fluorescence micrograph of magnetic beads immobilized with fluorescent liposomes in the Examples.
[Figure 3] It is a graph showing the immobilization efficiency of fluorescent liposomes of magnetic beads and gel beads in the Examples.
[Figure 4] It is a graph showing the relationship between the amount of cholesterol extracted from magnetic beads (fluorescence intensity) and the HDL concentration used in the Examples.
[Figure 5] It is a graph showing the correlation between the CEC evaluation method of the present invention and the CEC evaluation method using ILG.
[Figure 6] It is a graph showing the storage stability of immobilized liposome-bound magnetic beads in the Examples.
[Figure 7] It is a graph showing the washing effect of magnetic beads and the effect of blank correction before CEC measurement in the Examples.
[Figure 8] It is a graph showing the results of a CEC linearity test using diluted HDL samples in the Examples.
[Figure 9] It is a graph showing the correlation of CEC between the ILM method and ILG method using serially diluted HDL and BDS.
[Figure 10] It is a graph showing the relationship between CEC and HDL-C in BDS of healthy subjects according to the ILM method, and the correlation with the ILG method.

### Mode for Carrying Out the Invention

The method for measuring the cholesterol efflux capacity of HDL in the embodiments of the present invention will be explained.

The measurement method of the present embodiment is characterized in the use of magnetic beads immobilized with liposomes comprising at least partially labeled cholesterol (hereinafter, "at least partially labeled cholesterol" may be referred to as "labeled cholesterol" or simply "cholesterol"). When an HDL-containing sample to be measured is brought into contact with the magnetic beads in a solution, labeled cholesterol is extracted from the liposomes immobilized on the magnetic beads due to the ability of HDL to extract cholesterol, and the extracted labeled cholesterol is taken up by HDL and moves into solution. Therefore, the ability of HDL to extract cholesterol can be measured by measuring the labeling intensity of the solution and measuring the concentration of labeled cholesterol incorporated into HDL in the solution.

Here, in order to measure the amount of labeled cholesterol in a solution, it is necessary to separate the magnetic beads in the solution. In the previously reported CEC evaluation method using gel beads (ILG) for immobilizing cholesterol (Non-Patent Documents 4 and 5), there is the problem that centrifugation is required to separate the gel beads, making it difficult to install on an automatic analyzer. On the other hand, in the method of the present embodiment, since the magnetic beads can be separated using the magnetic force of magnets or the like, automation is easy if an analyzer capable of manipulating magnetic beads is used and they are suitable for implementation into routine clinical examinations.

In addition, when the magnetic beads are separated magnetically, compared to the separation of the gel beads by centrifugation, the process is shorter and the separation is more reliable, and further since no centrifugation is required, there is the advantage that the HDL in the solution can be stably separated.

In addition, unlike the cholesterol uptake capacity evaluation method of using an antibody in the previous report (Non-Patent Document 3), in the method for measuring the cholesterol efflux capacity of the present embodiment, the ability of free cholesterol in a solution to bind to HDL is not evaluated, but rather the ability of HDL to extract cholesterol is directly assessed. Therefore, the method for measuring cholesterol efflux capacity of the present embodiment is more suitable for evaluating the cholesterol efflux capacity *in vivo.*

In addition, the method for measuring cholesterol efflux capacity in the present embodiment does not use an antibody, and therefore it is of low cost.

Each step of the method for measuring the cholesterol efflux capacity of the present embodiment (hereinafter also referred to as "CEC measurement method" or "CEC evaluation method") will be described in more detail below.

### <Step of preparing magnetic beads on which cholesterol is immobilized>

In the CEC measurement method of the present embodiment, first, magnetic beads on which cholesterol is immobilized are prepared.

### (Cholesterol)

In the CEC measurement method of the present embodiment, it is preferred that at least partially labeled cholesterol is complexed with liposomes and immobilized on magnetic beads.

Liposomes are preferred particles for CEC measurements because they can contain labeled cholesterol and HDL can extract cholesterol. In addition, since liposomes composed of phospholipids and cholesterol have a structure similar to that of cell membranes and are similar to the *in vivo* environment, they can more accurately reflect the efflux capacity *in vivo.*

Further, in one embodiment, by including cholesterol in the liposome, there is also an advantage that the cholesterol can be efficiently immobilized on the magnetic beads by a simple method such as freezing and thawing, as will be described later.

As used herein, cholesterol-containing liposomes refer to liposomes in which cholesterol molecules are embedded on the membrane surface or in the membrane of the liposomes.

The liposomes in the present embodiment are not particularly limited, and include small unilamellar vesicles (SUV, average particle size of 100 nm or less), large unilamellar vesicles (LUV, average particle size of 100-500 nm), and multilamellar vesicles (MLV, average particle size of 200 to 1000 nm), but the average particle size is generally 10 to 300 nm, preferably 20 to 100 nm, more preferably about 50 nm, and such liposomes are preferred.

Cholesterol used in the preparation of liposomes may be cholesterol having a natural structure or one having a modified structure.

When the cholesterol contained in the liposomes immobilized on the magnetic beads is extracted by the HDL to be measured, it moves into the solution in the state of being incorporated into the HDL. The ability of HDL to extract cholesterol can be evaluated by measuring the amount (concentration) of cholesterol in a solution after separation of magnetic beads (In the present specification, the solution after separation of magnetic beads is sometimes referred to as "supernatant".). Therefore, in the measurement method of the present embodiment, at least partially labeled cholesterol is used to measure the cholesterol concentration in the supernatant.

Methods for labeling cholesterol include, but are not limited to, fluorescent substances, chromogenic substances, luminescent substances, radioactive substances, and stable isotopes. From the perspective of introducing into routine examination, fluorescent substances, chromogenic substances and luminescent substances are preferable, and fluorescent substances are more preferable.

As the labeled cholesterol, a commercially available one may be used as appropriate.

The phospholipid used for preparing liposomes is not particularly limited, but one or more of various phospholipids such as egg yolk lecithin, soybean lecithin, and hydrogenated soybean lecithin can be used.

The method for manufacturing the cholesterol-containing liposome is not particularly limited, and it can be manufactured by a known production method using one or more phospholipids and at least partially labeled cholesterol.

Examples of methods for preparing cholesterol-containing liposomes include those described in Yoshimoto et al., Biotechnol. Prog. 2006, 22, 459-464.

The ratio of cholesterol to phospholipid can be selected as appropriate, but it is for example, phospholipid: cholesterol = 50:1 to 1:2, preferably 20:1 to 1:1, more preferably 10:1 to 2:1 (weight ratio).

### (Magnetic beads)

The magnetic beads used in the CEC measurement method of the present embodiment are not particularly limited as long as they can immobilize the above-described cholesterol-containing liposomes, and examples include, in addition to particles made of magnetic materials, particles containing magnetic materials, such as composite particles of magnetic materials with organic polymer compounds, inorganic compounds, biopolymers, and the like.

Magnetic materials that constitute the magnetic beads or are contained in the magnetic beads include iron oxide (Fe₃O₄, γ-Fe₂O₃), various ferrites (magnetic ceramics), and metals such as iron, manganese, nickel, cobalt, chromium, alloys thereof, and the like.

Examples of composite particles comprising a magnetic material include, for example, particles of an organic polymer compound in which a magnetic material is dispersed, or particles having a core (core portion) consisting of or comprising a magnetic material and a shell (film) having an organic polymer compound or an inorganic compound.

The shape of the magnetic beads is not particularly limited, and may be spherical, substantially spherical, flattened spherical, amorphous, etc., but spherical or substantially spherical is preferable from the viewpoint of dispersibility. Further, the size of the magnetic beads is also not particularly limited, but can be, for example, 5 to 400 µm, preferably 10 to 200 µm, and more preferably 20 to 100 µm.

In one embodiment, the magnetic beads are preferably porous magnetic particles.

As described later, the porous magnetic particles can be freeze-thawed so that cholesterol can be efficiently immobilized in a simple manner.

The pore size of the porous magnetic particles can be appropriately selected according to the size of the liposomes to be immobilized, and for example, it can be 100 nm or more, preferably 300 nm or more, more preferably 500 nm or more, and 5 µm or less, preferably 2 µm or less, more preferably 1 µm or less. When the pore diameter (minor diameter) of the porous magnetic beads is 100 nm or more, the liposomes can easily enter the pores; and if it is 5 µm or less, the liposomes can easily remain inside the pores after the freeze-thaw process described below and can be immobilized efficiently.

Examples of magnetic porous particles include, but are not limited to, magnetic ceramics (ferrite) particles, and composite particles such as magnetic silica in which a magnetic material is coated with a porous substance such as silica.

Other magnetic beads include magnetic beads that have a surface structure that allows liposomes to bind, and such.

### (Immobilization on magnetic beads)

The method for immobilizing cholesterol-containing liposomes on magnetic beads is not particularly limited, and can be appropriately selected in consideration of the type of magnetic beads or such.

For example, there is a method in which liposomes are brought into contact with magnetic beads having a surface structure to which liposomes easily bind in an appropriate medium such as an aqueous medium, and the liposomes are adsorbed to the magnetic beads.

In particular, the present inventors have found that when the above-described porous magnetic particles are used as magnetic beads, the liposomes that contain cholesterol can be efficiently immobilized on magnetic beads by a simple method of repeating freeze-thaw cycles of a solution comprising the cholesterol-containing liposomes and the magnetic beads.

Specifically, magnetic beads are dispersed and mixed in a liposome suspension containing cholesterol, and for reaction, the mixture is kept still or stirred at 10 to 50°C, preferably at room temperature (20 to 30°C) for 5 minutes to 16 hours, preferably 10 minutes to 2 hours. As a medium for the liposome suspension (hereinafter also referred to as "suspension medium"), an aqueous medium such as water, physiological saline, various buffer solutions or such can be used. After that, the reaction solution is subjected to a freeze-thaw cycle, for example, a freeze-thaw cycle of freezing (e.g., -60°C or lower) and thawing (e.g., water bath / room temperature) 3 times or more, preferably 5 times or more, and 20 times or less, preferably 15 times or less to immobilize the liposomes on the magnetic beads.

The reason why cholesterol-containing liposomes can be efficiently immobilized on magnetic beads by such a process is that by mixing and reacting porous magnetic beads having an appropriate pore size with a cholesterol-containing liposome suspension, the liposomes enter the pores of the porous magnetic beads and when they are repeatedly frozen and thawed in this state, the liposomes increase in size and become clogged inside the pores, and the liposomes can be efficiently immobilized to the magnetic beads.

For the ratio of the cholesterol-containing liposomes to the magnetic beads, the cholesterol-containing liposome suspension to magnetic beads can be in the range of 20:1 to 20:20, preferably 10:1 to 10:6, more preferably 7:2 to 7:3 (mL:g). In addition, the liposome (phospholipid and cholesterol) content: magnetic beads can be in the range of 1:50 to 1:1000, preferably 1:100 to 1:500, more preferably 1:150 to 1:300 (g:g). With such a range, it is possible to analyze the solution after separation from the magnetic beads, that is, to evaluate the ability of the HDL-containing sample to extract cholesterol. Note that the above ratio may be appropriately adjusted in consideration of the phospholipid used in the liposome, the type of magnetic beads, and the like.

Magnetic beads on which cholesterol-containing liposomes prepared in this manner are immobilized (hereinafter also referred to as "ILM: immobilized liposome-bound magnetic beads") can be stored in a suspension state after purification. As the suspension medium for the ILM suspension, the same medium as the medium for the liposome suspension described above can be used.

The magnetic beads on which cholesterol-containing liposomes thus prepared are immobilized are stable in a cool and dark place (usually at 4°C or below, and in some cases preferably under light shielding), and are suitable for use in routine clinical examinations.

The storage stability of magnetic beads on which cholesterol-containing liposomes are immobilized can be evaluated by the change in labeling substance intensity in the suspension medium (for example, the supernatant) of the ILM suspension. For example, if the storage stability of the ILM is good, the amount of labeling substance (for example, fluorescently labeled cholesterol) liberated from the magnetic beads into the suspension medium during storage is small. In one embodiment, the labeling intensity (for example, fluorescence intensity) of the suspension medium taken from the ILM suspension does not significantly increase (does not substantially change) for at least 10 days, preferably at least 20 days, more preferably at least 30 days, even more preferably at least 40 days under the desired storage conditions (for example, at 4°C).

In the present specification, the magnetic beads on which cholesterol-containing liposomes are immobilized are sometimes simply referred to as "magnetic beads on which cholesterol is immobilized."

### <Step of contacting cholesterol-immobilized magnetic beads with HDL-containing sample to be measured>

Cholesterol is extracted from the liposomes immobilized on the magnetic beads by bringing the magnetic beads onto which the cholesterol-containing liposomes are immobilized, as prepared as described above, into contact with the HDL-containing sample to be measured in a solution.

The HDL-containing sample to be measured in the measurement method of the present embodiment is derived from mammals (for example, humans, bovines, pigs, horses, dogs, cats, sheep, goats, rabbits, hamsters, guinea pigs, mice, rats, and such), and an isolated HDL-containing sample derived from human is preferred.

As the HDL-containing sample, blood samples such as whole blood, serum, and plasma, apoB-deleted serum (BDS), purified HDL, and the like can be used.

HDL can be classified into a plurality of subclasses according to size, density, charge, composition of apolipoproteins, or such, but the measurement method of the present embodiment can also be used for measuring the cholesterol efflux capacity of subclasses of HDL (HDL₂ , HDL₃ , apoE-containing HDL , homocysteine-modified HDL, and such). Purified HDL and its subfractions can be purified using known methods such as ultracentrifugation.

ApoB deleted serum (BDS) is obtained by removing the lipoprotein fraction that contains apolipoprotein B (apoB) by the polyethylene glycol (PEG) precipitation method. ApoB-depleted serum has been reported in the present inventors' previous report (Non-Patent Document 5) to be correlated with the CEC of purified HDL, and is easier to prepare than purified HDL, and therefore it can be suitably used as a test sample for clinical examination.

As the HDL-containing sample to be measured, the above-mentioned blood sample, apoB-depleted serum (BDS), or purified HDL diluted with an aqueous medium can be used. The aqueous medium includes water, physiological saline, various buffers (phosphate buffered saline (PBS), Tris-HCl, etc.), and the like. The concentration of the HDL-containing sample can be appropriately selected in consideration of the type of sample, the scale of the reaction system, or such. In the case of purified HDL, the final total cholesterol concentration is 0.1 to 100 mg/dL, preferably 1 to 50 mg/dL. In the case of BDS, the final serum concentration can be adjusted to 0.1 to 10%, and preferably 1 to 5%.

The reaction between the cholesterol-immobilized magnetic beads and the HDL-containing sample to be measured is, for example, incubation at 0 to 50°C, preferably 10 to 40°C, more preferably room temperature (20 to 30°C), for 1 to 72 hours, preferably 4 to 48 hours, more preferably 8 to 24 hours. Further, from the viewpoint of shortening the measurement time, the reaction time may be 1 to 16 hours, preferably 1 to 8 hours, and more preferably 2 to 4 hours. During this time period, the reaction solution may be kept still, or may be stirred or shaken.

Further, the magnetic beads on which cholesterol is immobilized may be washed before the CEC measurement. By performing the washing operation, it is possible to reduce the measurement of the labeling substance released from the magnetic beads. The washing operation can be performed by recovering the ILM from the ILM suspension by centrifugation or using a magnet or the like, and resuspending the recovered ILM in a desired volume of the suspension medium.

Also, in one embodiment, blank correction may be performed instead of the washing operation, as shown in the examples below. By performing blank correction without the washing operation, it is possible to perform CEC measurement with the same accuracy as when the washing operation is performed. Blank correction can be performed, for example, by measuring CEC using a dilution medium of an HDL-containing sample (the aqueous medium described above) as a blank sample instead of the HDL-containing sample to be measured in the CEC measurement, and subtracting that value as a blank from the measured CEC value.

### <Step of evaluating cholesterol efflux capacity by separating magnetic beads and measuring cholesterol concentration in solution after magnetic bead separation>

When the magnetic beads having cholesterol immobilized thereon are reacted with the HDL-containing sample to be measured as described above, cholesterol is extracted from the liposomes immobilized on the magnetic beads by the action of HDL in the solution, and then it will be moved into the solution in the entrapped state.

To measure this extracted cholesterol, the magnetic beads are first separated. Separation of the magnetic beads may be performed using a magnetic force such as a magnet. As described above, the CEC measurement method of the present embodiment does not require centrifugal separation, and thus can be easily applied to an automatic analyzer.
In addition, since magnetic beads enable rapid separation, measurement time can be shortened. Further, gel beads may float and contaminate the supernatant when the supernatant is collected after centrifugation. However, with magnetic beads, the magnetic beads can be reliably separated by immobilization using magnetism.

Furthermore, since centrifugal operation is not required, there is also the advantage of being able to separate stably.

Further, a centrifugal separation operation may be performed in addition to separation by magnetic force such as a magnet, or in some cases instead of separation by magnetic force. As described above, the gel beads used in the ILG method have a problem that light gel beads tend to scatter when recovering the supernatant after centrifugation and are affected by error, but the magnetic beads (ILM) of the present embodiment are advantageous in that they can be easily separated using a magnet, and also because they are heavy and can be strongly attracted by a magnet, they do not fly around like gel beads, making it easier to process, and thus more accurate measurement is possible.

Next, the concentration of the cholesterol in the supernatant obtained by separating the magnetic beads is measured. The cholesterol concentration in the supernatant can be determined by measuring the signal derived from the cholesterol labeling substance in the supernatant by a method according to the type of labeling.

The ability to extract cholesterol can be quantified by determining the amount of cholesterol extracted from the measured cholesterol concentration in the supernatant and converting it to per unit amount of HDL (per 20 mg HDL protein/mL or per 1 mg HDL-C/dL) (by HDL to HDL comparison). In addition, by using a specific reference serum or apoB-depleted serum (BDS) sample as a reference sample, the CEC represented by the amount of cholesterol extracted by HDL is normalized and/or the influence of the difference between lots of magnetic beads can also be excluded.

As shown in the Examples below, in the CEC measurement method using the ILM of the present embodiment, linearity is recognized in a wide HDL concentration range, and highly accurate CEC measurement can be performed. In one embodiment, it is possible to measure CEC in a concentration range of 50 to 200 mg/dL, preferably 30 to 220 mg/dL in terms of the serum HDL cholesterol concentration of the subject, and therefore the specimens of the healthy subjects and patients can be sufficiently measured.

In addition, the CEC measurement method using the ILM of the present embodiment has high reproducibility as shown in the Examples below. For example, in the CEC measurement method of the present embodiment, the coefficient of variation (CV%) of the CEC measurement values of at least 10 replicate measurements, preferably at least 20 replicate measurements of the same sample on the same day is 10% or less, preferably 7% or less. Also, the coefficient of variation (CV%) of the CEC measurement values of the same sample over at least 10 days, preferably at least 20 days is 10% or less. As described above, by the CEC measurement method of the present embodiment, CEC measurement can be performed with high reproducibility as compared with the conventional cell method.

Furthermore, as shown in the Examples below, in the CEC measurement method of the present embodiment, even if the HDL-C concentration is of the same degree, the CEC measurement value differs if the cholesterol efflux capacity of HDL is different. As described above, the CEC measurement method of the present embodiment can accurately evaluate differences in the ability of HDL to extract cholesterol. Therefore, in one aspect of the present invention, the results of the CEC measurement method of the present embodiment can be used to obtain information on the conditions related to the ability of a subject to extract cholesterol or the risks thereof. In addition, by comparing and/or accumulating data obtained from HDL-containing samples (apoB-depleted serum (BDS) samples, purified HDL samples, or such) of healthy subjects and patients, it is possible to define a threshold value or reference range of the cholesterol efflux capacity for the conditions related to cholesterol efflux capacity or risks thereof, as well as to determine whether a subject's cholesterol efflux capacity is within the reference range.

Conditions related to the cholesterol efflux capacity include, for example, dyslipidemia, atherosclerosis, familial cholesterolemia, inflammatory diseases (in particular, inflammatory diseases associated with the decrease of cholesterol efflux capacity, or such), diabetes, and risk of developing a cardiovascular disease event.

The measurement method of the present embodiment is an *in vitro* method that can be performed in a cell-free system.

Furthermore, one aspect of the present invention relates to a test method for evaluating the ability of HDL to extract cholesterol using magnetic beads on which cholesterol is immobilized.

Furthermore, one aspect of the present invention relates to a test method for evaluating the ability of HDL to extract cholesterol using magnetic beads on which cholesterol is immobilized.

Furthermore, one aspect of the present invention relates to a reagent kit for measuring the cholesterol efflux capacity used in the CEC measurement method of the present embodiment.

The CEC measurement reagent kit of the present embodiment comprises at least magnetic beads on which cholesterol is immobilized, or magnetic beads for use in manufacturing magnetic beads on which cholesterol is immobilized. The CEC measurement reagent kit of the present embodiment further comprises cholesterol for use in manufacturing magnetic beads having cholesterol immobilized thereon, various reagents for use in preparing an HDL-containing sample, and any reagents used in the CEC measurement method of the present embodiment, such as a medium for reaction of magnetic beads on which cholesterol is immobilized with an HDL-containing sample, and various reagents for use in measuring the amount of labeled cholesterol, as well as containers and the like for storage and reaction of these reagents.

One aspect of the present invention further relates to use of magnetic beads having cholesterol immobilized thereon in a method for measuring CEC, an inspection method for evaluating CEC, and a test method for evaluating CEC, as well as their use in manufacturing a reagent kit for CEC measurement.

According to the method for measuring CEC, the inspection method for evaluating CEC, the test method for evaluating CEC, and the CEC measurement reagent kit of the present embodiment, the cholesterol efflux capacity of HDL can be evaluated and/or information on the conditions related to the cholesterol efflux capacity of a subject in a cell-free system and risks thereof can be acquired by a simple process in which a reagent containing magnetic beads immoilized with cholesterol is brought into contact with a sample containing HDL to be measured under the desired conditions, the magnetic beads are separated, and the cholesterol concentration in the supernatant or solution after separation of the magnetic beads is measured.

Therefore, the method for measuring CEC, the inspection method for evaluating CEC, the test method for evaluating CEC, and the CEC measurement reagent kit according to the present invention can be easily applied to automatic analyzers, and they are suitable for application to routine examinations in clinical practice.

All documents referred to in the present specification are hereby incorporated by reference in their entirety.

The examples of the present invention described below are for illustrative purposes only and do not limit the technical scope of the present invention.

### Examples

### <Method>

### (1) Preparation method of immobilized liposome-bound beads

### (Preparation Example 1)

### Preparation of immobilized liposome-bound magnetic beads

Egg lecithin (10.6 mg) and cholesterol (2.3 mg) were dissolved in 6 mL of chloroform, and 30 µL of 0.5 mM fluorescence-labeled cholesterol (4,4-difluoro-4-bora-3a , 4a-s-indacene-labeled cholesterol, Avanti Polar Lipids) was added to the solution. The lipid film formed under nitrogen gas was dissolved in ether and the solvent was removed by evaporation. After this step was performed twice, the lipid film was completely dried under nitrogen gas and suspended in 7 mL of 10 mM Tris-HCl (pH 7.4) containing 150 mM NaCl and 1 mM EDTA-2Na (Buffer A) (liposome suspension). After that, 2.45 g of magnetic beads (core-shell ferrite powder, MTFH-35, POWDERTECH; particle size: 40.2 µm and pore size: 0.8 µm) were added to the liposome suspension and allowed to react at room temperature for 30 minutes. The mixture was subjected to 3, 5, 7, and 9 cycles of freezing (-80°C) and thawing (room temperature, in water) repeatedly to induce immobilization of the liposomes into the beads. Finally, it was washed 5 times with Buffer A and resuspended in 5 mL of Buffer A. The bead suspension was stored in the dark at 4°C.

### (Comparative Preparation Example 1)

### Preparation of ILG (Immobilized Liposome-Binding Gel Beads)

The preparation of ILG (immobilized liposome-bound gel beads) was performed in the same manner as in Example 1, with the exception that 0.35 g of dry gel beads (Sephacryl S-300 gel beads, GE Healthcare) were added to the liposome suspension instead of 2.45 g of magnetic beads, and 7 cycles of freezing and thawing were performed.

### (2) Recovery of BDS by precipitation method

A 20% polyethylene glycol solution (200 mM glycine buffer; pH 7.4) was added to the serum at a ratio of 100:40, and after incubation (20 min, room temperature) and centrifugation (10,000 rpm, 30 min, 4°C), the supernatant was collected as BDS.

### (3) Measurement of cholesterol efflux capacity (CEC)

To 100 µL suspension of immobilized liposome-bound magnetic beads containing fluorescently labeled cholesterol (Preparation Example 1) or 100 µL suspension of immobilized liposome-bound gel beads (Comparative Preparation Example 1), 150 µL of diluted HDL (measurement sample) or BDS (reference sample) was added, so that the final total cholesterol concentration became 4 to15 mg/dL (in the case of HDL) and the final serum concentration became 2% (in the case of BDS), respectively. After incubation (16h, 30°C) under light-shielded conditions, the immobilized liposome-bound magnetic beads were separated from the reaction solution using a magnet and the immobilized liposome-bound gel beads were separated using centrifugation, and the fluorescence intensities (Ex: 485 nm, Em: 538 nm) of the resulting 75 µL supernatants were measured.

In addition, unless otherwise specified, the CEC measurement was performed in the same manner as in the present example also in the examples after (4).

### <Results>

### (1) Immobilization of liposomes by freezing and thawing

In Preparation Example 1, in order to confirm that the fluorescently labeled cholesterol-containing liposomes were immobilized on the magnetic beads, the supernatant before washing was collected after freezing and thawing. The fluorescence intensity in the beads was evaluated by comparing the fluorescence intensity of the liposome suspension before the reaction with the fluorescence intensity of the magnetic beads and the supernatant after the reaction.

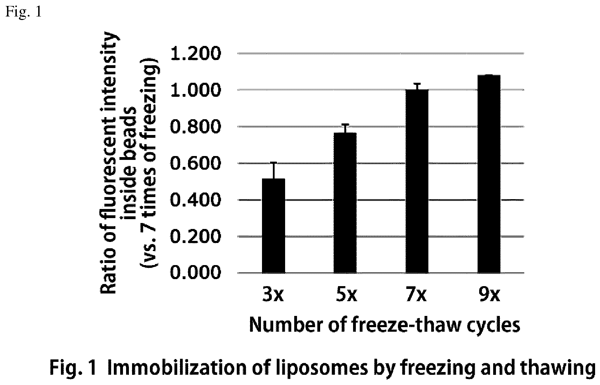

Fig. 1 shows a comparison of the amounts of liposomes immobilized by changing the number of times of freezing. The results are shown as the ratio of the fluorescence intensity in the beads relative to 7 freeze and thaw cycles. Liposomes were immobilized by repeated freezing and thawing.

### (2) Fluorescence photograph of immobilized liposome-bound magnetic beads

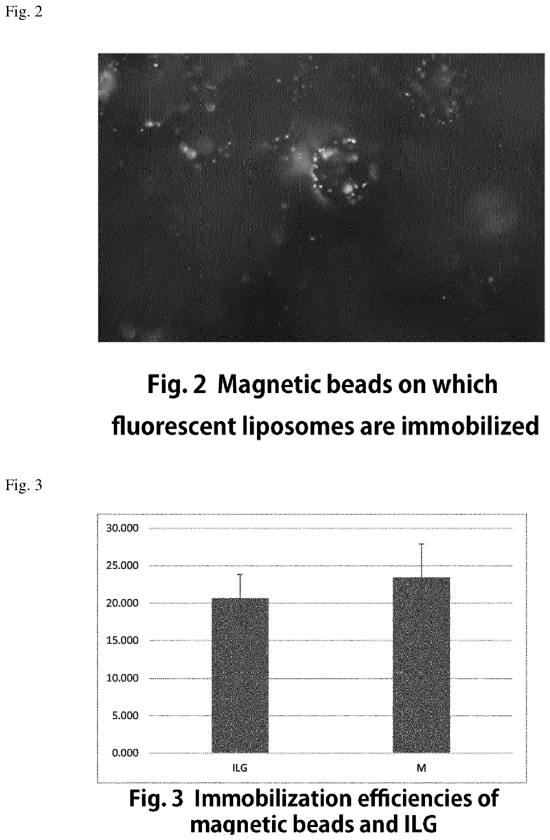

In Preparation Example 1, the fluorescently labeled cholesterol-containing liposomes were immobilized on magnetic beads, and then after washing, the immobilized magnetic beads were observed under a fluorescence microscope (Fig. 2). Fluorescence was observed inside the magnetic beads, confirming that the liposomes were immobilized.

### (3) Comparison of liposome immobilization efficiency between magnetic beads and gel beads

Fig. 3 shows the result of comparing the liposome immobilization efficiency between the magnetic beads of the present invention and the conventional gel beads.

In Fig. 3, "M" is the amount of immobilized liposome-bound magnetic beads prepared (2 times of preparation (3 tubes each time, 6 tubes in total)) in the same manner as in Preparation Example 1 (5 times of freezing and thawing) . "ILG" shows the amount of immobilized liposome-bound gel beads prepared (2 times of preparation on average (2 tubes each time, 4 tubes in total)) in the same manner as in Comparative Preparation Example 1 (7 times of freezing and thawing). It was confirmed that liposomes could be immobilized on magnetic beads with an efficiency equal to or higher than that of gel beads.

### (4) Cholesterol efflux capacity by immobilized liposome-bound magnetic beads

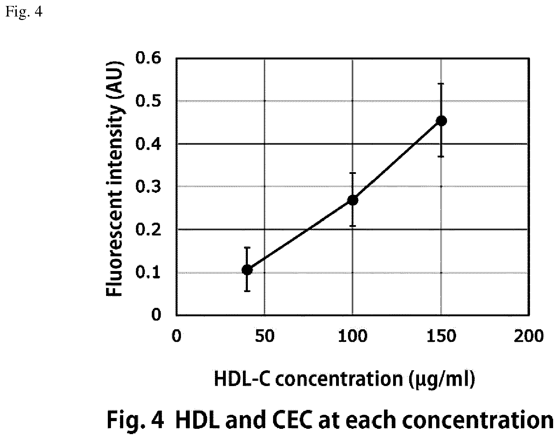

Using the magnetic beads of Preparation Example 1 which had been frozen five times, the ability to extract cholesterol from HDL at different concentrations was evaluated. The ability to extract fluorescent cholesterol increased in an HDL concentration-dependent manner (Fig. 4).

### (5) Correlation with the conventional method

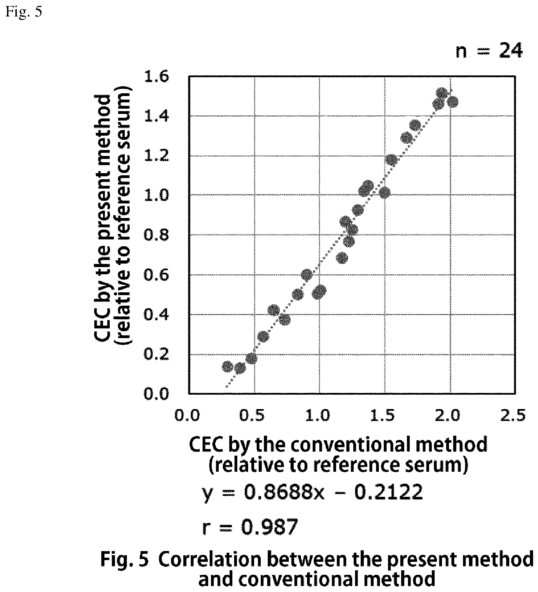

Using various HDL concentrations (24 series, 1 to 15 mg/mL), the CEC correlation between the conventional method using ILG and the method of the present invention using magnetic beads was investigated. (Fig. 5). The CEC of each sample was normalized with the CEC measured using the same reference serum. The method of the present invention showed good correlation with the conventional method (r = 0.987). In addition, it is reported that the conventional method using ILG shown in the present example has a high correlation with the conventional CEC evaluation method using cultured cells (Non-Patent Document 4, r = 0.932).

### (6) Storage stability of immobilized liposome-binding magnetic beads (ILM)

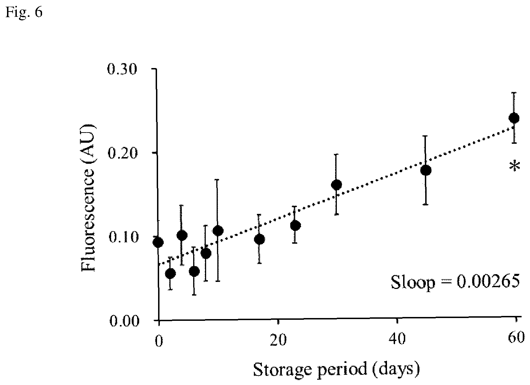

Fluorescent cholesterol-containing liposomes were immobilized on magnetic beads in the same manner as the magnetic beads that had been frozen five times in Preparation Example 1. The ILM was stored at 4°C in the dark for 0 to 60 days, and the fluorescence intensity of the supernatant was measured for each storage period. Although the fluorescence intensity of the supernatant (reflecting the amount of fluorescent cholesterol-containing liposomes released during storage) slightly increased over 60 days, no significant difference was observed up to 45 days compared to 0 days (Fig. 6). Three tubes were stored for each day, and each fluorescence intensity was measured in triplicate. *p < 0.05.

### (7) Effect of washing magnetic beads before CEC measurement and effect of blank correction

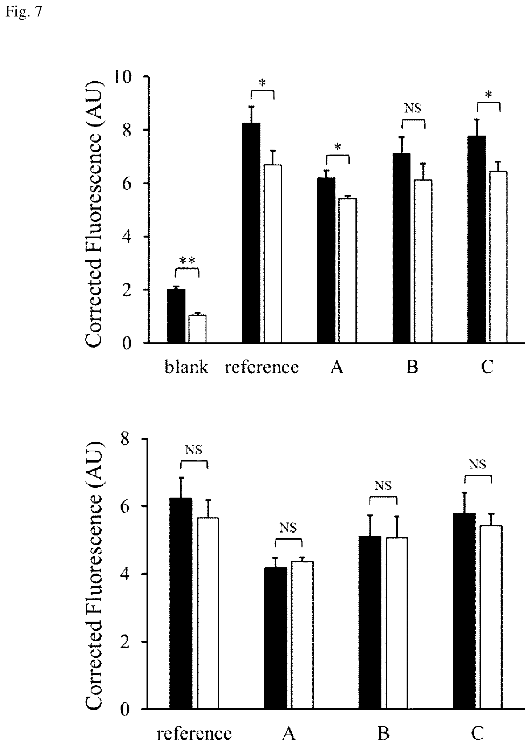

Fluorescent cholesterol-containing liposomes were immobilized on magnetic beads in the same manner as the magnetic beads that had been frozen five times in Preparation Example 1. Using this ILM, the CEC of the buffer alone (blank), reference serum and serum samples A, B and C were measured. During the CEC measurement, the CEC was compared between the operation of washing the magnetic beads with a buffer solution (white bar graph) and not washing them (black bar graph) (Fig. 7, upper panel). The washing operation was carried out by repeating the operation of collecting the ILM using a magnet and resuspending it in the same volume of Buffer A twice. A significant difference in CEC was observed in most samples (other than serum B) depending on whether or not the magnetic beads were washed (*p < 0.05, **p < 0.01). This is because the fluorescence intensity released from the magnetic beads is scaled if not washed. On the other hand, when the CEC values of the reference serum, serum A, B, and C were subtracted by the blank value (blank correction) (Fig. 7, lower panel), no significant difference was observed between the presence and absence of the washing operation. In other words, CEC can be measured by performing blank correction without washing.

### (8) Linearity test of CEC using diluted HDL and BDS samples

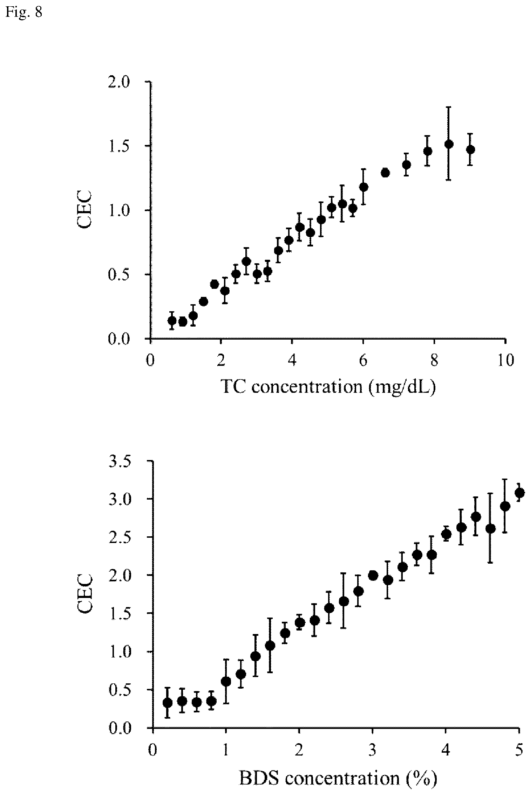

After 24 serial dilutions of HDL obtained in the range of 0.6 to 9.0 mg cholesterol/dL by ultracentrifugation, the CEC of each diluted HDL was measured. As a result, linearity was observed from 1.2 to 7.8 mg/dL (Fig. 8, upper panel, TC: total cholesterol). Similarly, when BDS was diluted in the range of 0.2 to 5.0% and measured, linearity was observed up to the range of 1.0 to 5.0% (Fig. 8, lower panel). When converted to serum HDL cholesterol concentration, this means that approximately 34 to 223 mg/dL can be measured, and it is possible to measure the specimens of healthy subjects and patients sufficiently.

### (9) CEC correlation between the ILM method and the ILG method using serially diluted HDL and BDS

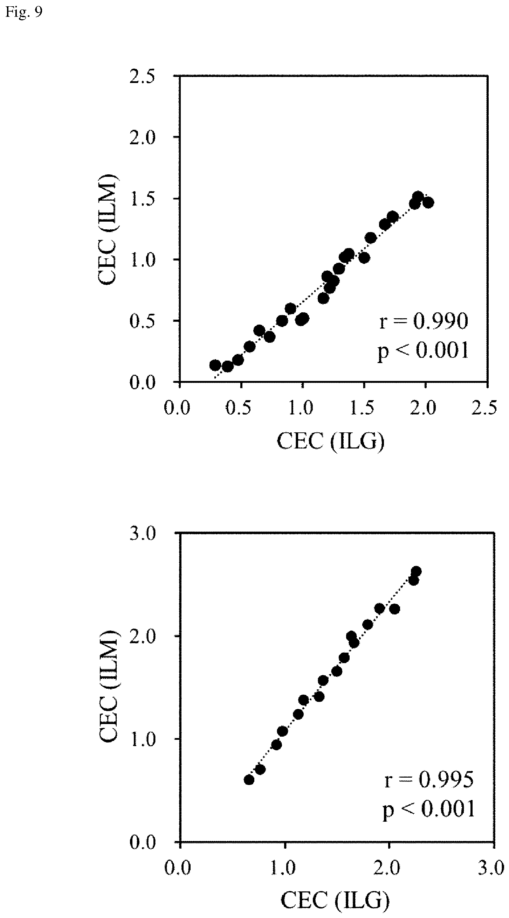

A correlation test between the ILG method and ILM method was performed using samples in the concentration range where linearity was confirmed in (8) above. (Fig. 9 upper panel: HDL, Fig. 9 lower panel: BDS). A good correlation was observed for both.

### (10) Relationship between CEC and HDL-C in BDS of healthy subjects by the ILM method, and correlation with the ILG method

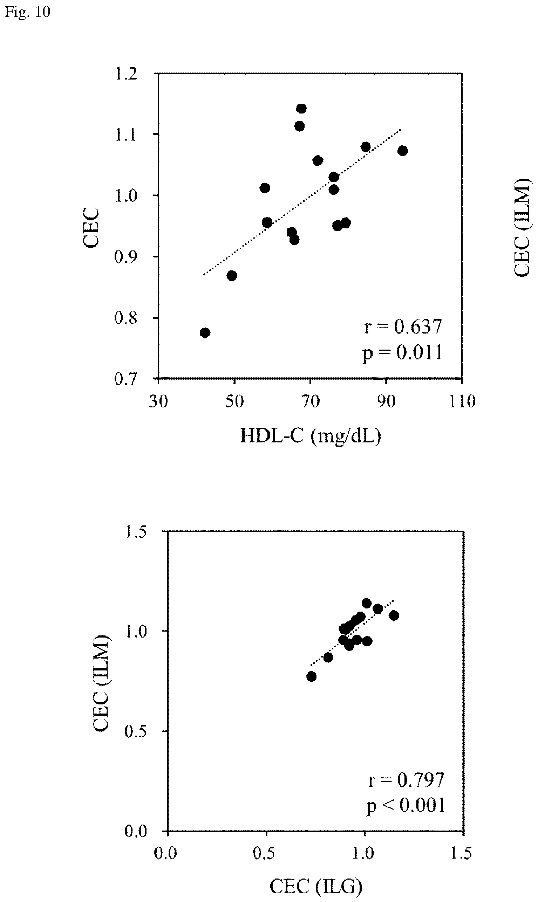

CEC in the BDS of 15 healthy subjects was measured by the ILM method (in triplicate) and the correlation with their respective HDL-C concentrations was confirmed. A positive correlation was observed between CEC and HDL-C, but some samples had different CEC even at similar HDL-C concentrations, which may reflect differences in HDL capacity (Fig 10, upper panel). The ILM method and ILG method also showed good correlation (Figure 10, lower panel).

### (11) Reproducibility

When the CEC of BDS with three different concentrations was measured in 20 replicates on the same day by the ILM method, the CV% was within 7% in all cases (Table 1: Samples 1-3). Moreover, when 3 types of BDS were similarly measured for 20 days, the CV% was within 10% (Table 1: Samples 4-6). When the conventional cell method was used, these CV% exceeded 10% (data not shown), indicating better reproducibility of the ILM method of the present invention compared to the conventional method.

**Table 1**

| Reproducibility | | | | | |
|---|---|---|---|---|---|
| | Within-Dav | | | Between-Day | |
| | CEC (Mean ±SD) | CV (%) | | CEC (Mean ±SD) | CV (%) |
| Sample 1 | 0.756 ± 0.044 | 5.8 | Sample 4 | 0.806 ± 0.063 | 7.7 |
| Sample 2 | 0.839 ± 0.051 | 6.1 | Sample 5 | 0.873 ± 0.080 | 9.2 |
| Sample 3 | 0.952 ± 0.063 | 6.6 | Sample 6 | 0.872 ± 0.080 | 9.2 |

### Industrial Applicability

From these results, the CEC evaluation method of the present invention using magnetic beads with immobilized cholesterol shows a high correlation with the conventional CEC evaluation method, and since CEC can be evaluated by a simple process that does not use cultured cells or centrifugation, it was shown that automation is easy and that it can be easily applied to routine clinical examinations.

## Claims

1. A method for measuring the cholesterol efflux capacity of HDL, wherein the method comprises:
preparing magnetic beads on which cholesterol is immobilized;
contacting the magnetic beads on which cholesterol is immobilized with an HDL-containing sample to be measured in a solution; and
separating the magnetic beads from the solution and measuring the amount of cholesterol in the solution after separation of the magnetic beads.

2. The method according to claim 1, wherein the magnetic beads are porous magnetic particles.

3. The method according to claim 1 or 2, comprising preparing magnetic beads on which cholesterol is immobilized, preparing liposomes comprising cholesterol, and freezing and thawing a solution comprising the liposomes and magnetic beads.

4. The method according to any one of claims 1 to 3, wherein at least part of the cholesterol immobilized on the magnetic beads is fluorescently labeled.

5. The method according to any one of claims 1 to 4 , which is a method for measuring the cholesterol efflux capacity of HDL, comprising:
preparing magnetic beads on which cholesterol is immobilized by preparing liposomes comprising at least partially labeled cholesterol, and freezing and thawing a solution comprising the liposomes and porous magnetic particles;
contacting the magnetic beads on which cholesterol is immobilized with an HDL-containing sample to be measured in a solution; and
separating magnetic beads from the solution and measuring the amount of cholesterol in the solution after magnetic bead separation based on the labeling intensity of the solution.

6. The method according to claim 5, wherein the cholesterol-immobilized magnetic beads have storage stability represented by the fact that the labeling intensity of a suspension medium collected from a suspension of the magnetic beads does not substantially change for at least 10 days.

7. The method according to any one of claims 1 to 6, wherein the magnetic beads are magnetic ceramic particles.

8. The method according to any one of claims 1 to 7, wherein the step of separating the magnetic beads from the solution does not include centrifugation.

9. The method according to any one of claims 1 to 8, further comprising performing blank correction by subtracting the measured value of cholesterol quantity in the blank sample from the measured value of cholesterol quantity in the HDL-containing sample to be measured.

10. The method according to claim 9, which does not include washing the magnetic beads on which cholesterol is immobilized before the step of contacting the magnetic beads on which cholesterol is immobilized with the HDL-containing sample to be measured in a solution.

11. The method according to any one of claims 1 to 10, wherein the HDL-containing sample to be measured is whole blood, serum, plasma, apoB-depleted serum, or a solution comprising purified HDL.

12. The method according to any one of claims 1 to 11, which is performed in a cell-free system.

13. The method according to any one of claims 1 to 12, which has a reproducibility represented by a coefficient of variation of 10% or less for cholesterol quantity measurements of the same sample over at least 10 days.

14. A reagent kit for measuring the cholesterol efflux capacity of HDL, comprising magnetic beads to which cholesterol is immobilized.

15. A method for obtaining information on a condition related to the cholesterol efflux capacity of a subject or risk thereof, comprising measuring the cholesterol efflux capacity of an HDL-containing sample derived from the subject using magnetic beads on which cholesterol is immobilized.
